# EUROPEAN PATENT SPECIFICATION

(11) **EP 4 548 091 B1**
(45) Date of publication and mention of the grant of the patent: **18.11.2026**
(21) Application number: 23738200.7
(22) Date of filing: 21.06.2023
(51) Int. Cl.: G01N 33/04, A01J 5/007, A01J 5/013, A01J 5/04, G01N 33/558

(54) **ARRANGEMENT FOR EVALUATING PERFORMANCE OF A MILK DOSING ARRANGEMENT AND/ OR A LATERAL FLOW STICK**
ANORDNUNG ZUR BEWERTUNG DER LEISTUNG EINER MILCHDOSIERANORDNUNG UND/ODER EINES QUERSTROMSTICKS
AGENCEMENT PERMETTANT D'ÉVALUER LES PERFORMANCES D'UN AGENCEMENT DE DOSAGE DE LAIT ET/OU D'UN BÂTONNET À FLUX LATÉRAL

(30) Priority: 29.06.2022 SE 2250805
(43) Date of publication of application: 07.05.2025
(73) Proprietor: DeLaval Holding AB, 147 21 Tumba (SE)
(72) Inventor: GUDMUNDSSON, Kristjan Freyr, 147 21 Tumba (SE); NOROUZI, Ehsan, 147 21 Tumba (SE)
(74) Representative: DeLaval International AB
(86) International application number: PCT/SE2023/050641
(87) International publication number: WO 2024/005692

(56) References cited:
- WO-A1-2017/144913
- WO-A1-2018/236271
- WO-A1-2020/251460

## Description

### TECHNICAL FIELD

This document discloses an arrangement according to claim 1. More particularly, an arrangement is disclosed, arranged to evaluate performance of a milk dosing arrangement and/ or a lateral flow stick.

### BACKGROUND

On an animal farm, it is important to keep the animals healthy in order to enhance milk/ meat production. It is very important, in particular on a diary farm, to inseminate animals at an optimal moment in order to successfully fertilise the animal, for efficiency reasons. In case the animal is not successfully inseminated, milk production is affected.

Several biomarker measurements may be made on the animal, such as e.g. measuring levels of progesterone, LDH (Lactate Dehydrogenase), BHB (Beta-Hydroxybutyrat) and urea. Thereby important information concerning e.g. heat detection and/ or pregnancy of the individual animal may be made (based on measured progesterone level), as well as mastitis (based on LDH) and ketosis (based on BHB). Also, the energy balance may be estimated (based on urea).

A milk analysis apparatus/ service module may be arranged to cooperate with a milk extracting arrangement, for regularly analysing milk samples of the animals, e.g., at or about the moment of a milking session. The milk analysis apparatus/ service module may extract a milk sample and provide it on a lateral flow stick/ lateral flow test strip/ dry stick. The milk may be diluted with a diluent, which also may be used to rinse the tubings between test sessions. The diluent may be provided in a separate liquid container.

The lateral flow sticks may be maintained in a cassette, for example on a carrier tape in the cassette as disclosed in document WO2018236271 or WO2020251460.

Thereby lateral flow sticks may be easily administrated at the farm by forwarding one lateral flow stick at the time. One lateral flow stick may typically be used for each test sample. The cassette may comprise a large amount of lateral flow sticks, yet the cassette with the lateral flow sticks has to be exchanged for a new one when all the lateral flow sticks have been consumed.

Thereby, a farmer/ operator is provided with important information concerning each individual animal. The biomarker such as progesterone may be measured of all individual animals at the farm with a high degree of automation.

However, a problem that may occur is that it is impossible or at least very difficult/ time consuming for the farmer to determine if the milk sample has been correctly applied, and/ or that the lateral flow stick functions. As the process is automated, no human supervision of each single test session is made.

An example of a problem that may occur is that no liquid sample (that may comprise extracted milk + diluent) has been applied on the sample pad of the lateral flow stick.

Another problem may be that the applied liquid sample does not comprise (a sufficient amount of) milk, but only/ mainly diluent.

The reasons may for example be that a tubing is clogged, a pump is dysfunctional, a mechanical damage of the needle that supplies the liquid sample to the lateral flow stick, etc.

Yet a problem that may occur is that at least some of the lateral flow sticks may not operate as intended.

The reasons may for example be inappropriate storage of the lateral flow sticks. High temperature, or otherwise inappropriate storage of the lateral flow sticks may cause the antibody treated gold particles of the conjugate pad to burn into the cellulose. The milk sample will then not release enough gold particles to get a reliable measurement. Other reasons may comprise damage caused by damp/ liquid of the lateral flow sticks, usage of non-authorised third-party-made (or piracy-made) lateral flow sticks of low quality, and/ or that the lateral flow sticks are too old, etc.

In case the lateral flow test is invalid due to these or similar problems, it may be impossible to make a correct biomarker measurement.

It would for these reasons be advantageous to find a way to ascertain that lateral flow tests performed on the lateral flow sticks are valid and reliable, preferably without putting an additional workload on the farmer for determining the validity.

### SUMMARY

It is therefore an object of this invention to solve at least some of the above problems and evaluate performance of a milk dosing arrangement and/ or a lateral flow stick, when determining presence of a biomarker in a milk sample extracted from an animal, by a lateral flow test.

According to a first aspect of the invention, this objective is achieved by an arrangement, in accordance with independent claim 1 which is arranged to evaluate performance of a milk dosing arrangement and/ or a lateral flow stick The arrangement comprises the lateral flow stick arranged to indicate a biomarker in a liquid sample comprising a milk sample extracted from an animal, by a lateral flow measurement. The biomarker may for example be progesterone.

The lateral flow stick comprises a sample pad for receiving the liquid sample; a conjugate pad comprising conjugate; an indication zone, arranged to indicate the biomarker of the liquid sample, based on the conjugate; and a porous membrane for receiving a capillary flow of the liquid sample from the conjugate pad, thereby forwarding conjugate dispersed into the liquid sample to the indication zone.

The arrangement also comprises the milk dosing arrangement, configured to obtain a milk sample from the animal; and apply the liquid sample comprising the milk sample to the sample pad of the lateral flow stick. In addition, the arrangement comprises a camera directed to capture an image of the lateral flow stick. Also, the arrangement comprises a controller, communicatively connected to the camera.

The controller is configured to generate an instruction to the camera, to capture an image of the lateral flow stick at a predetermined time period after a point in time when the liquid sample has been applied to the lateral flow stick. The controller is also configured to receive the captured image from the camera. In addition, the controller is configured to define a first area of the image of the lateral flow stick, substantially depicting a subsection of the porous membrane upstream the indication zone in the liquid flow direction of the applied liquid sample along the longitudinal extension of the lateral flow stick. Furthermore, the controller is configured to estimate the colour or grey scale tint of the conjugate pad on the first area by comparing the colour or grey scale tint of the first area with the colour or grey scale tint of the second area; and evaluate performance of the milk dosing arrangement and/ or the lateral flow stick, based on the comparison of the colour or grey scale tint of the first area with the colour or grey scale tint of the second area.

The image processing of the first area will result in a detection of an either successfully or unsuccessfully released conjugate into the liquid sample, and a functional capillary flow of the liquid sample with the conjugate through at least a part of the porous membrane.

It could thereby, already within some few seconds, be determined if the milk dosing arrangement and/ or the lateral flow stick are fully functional and the outcome of the lateral flow measurement will be reliable.

An automated surveillance of the functionality of the milk dosing arrangement and/ or the lateral flow stick is thereby obtained, leading to increased reliability of the lateral flow measurement and the milk dosing arrangement and/ or the lateral flow stick.

In an implementation of the arrangement according to the first aspect, the controller may be configured to perform the image processing by colour or grey scale analysis of the first area.

The conjugate comprised in the conjugate pad has a colour tint which is distinct from white, the typical colour of the lateral flow stick. In case of gold conjugate, the colour is typically red. In the colour or grey scale analysis, it could be determined if conjugate of the conjugate pad has been dispersed into the liquid sample and transported through at least a part of the porous membrane.

In another implementation of the arrangement according to the first aspect, or any implementation thereof, the controller may be configured to perform the image processing and evaluate performance of the milk dosing arrangement by comparing a colour or grey scale tint on the first area of the captured image with a reference colour or grey scale tint.

The reference colour or grey scale tint used for the comparison may be predetermined and stored in a memory device communicatively connected to the controller.

In another implementation of the arrangement according to the first aspect, or any implementation thereof, the reference colour or grey scale tint may be obtained from a first image of the first area captured before application of the liquid sample to the sample pad.

An advantage with using an image of the first area captured before application of the liquid sample is that a robust and thrust worthy reference is achieved, which is independent from for example a colour deviation of the porous membrane of different lateral flow sticks; or odd light conditions at the location of the arrangement. Thereby, also a very small deviation of lateral flow sticks over time is detectable and could be determined.

By the made comparison, it could in a reliable way be estimated if the conjugate has/ have been released from the conjugate pad (in the second area of the image) and transported to the porous membrane (in the first area of the image) by the liquid sample.

In another implementation of the arrangement according to the first aspect, or any implementation thereof, the predetermined time period after the point in time when the liquid sample is applied to the lateral flow stick is shorter than the time it takes for the liquid sample to propagate by capillary flow from the sample pad via the conjugate pad and the porous membrane to the indication zone of the lateral flow stick, in addition to incubation time before a result is indicated by the indication zone.

The time it takes for the liquid sample to propagate via the porous membrane to the indication zone and achieve the result of the lateral flow measurement may be at least two minutes, including incubation time. The predetermined time period mentioned above may only be some seconds, such as maybe 5-10 seconds. Thus, it could immediately after application of the liquid sample be detected that the milk dosing arrangement and/ or the lateral flow stick are not working properly. It is thereby enabled to repeat the lateral flow measurement with a new milk sample from the same animal on the sample pad of a new lateral flow stick.

In another implementation of the arrangement according to the first aspect, or any implementation thereof, the controller may be configured to repeatedly generate instructions to the camera to capture images of the lateral flow stick. The controller may also be configured to receive the captured images from the camera with a respective time stamp. The image processing and the evaluation of the performance of the milk dosing arrangement and/ or the lateral flow stick is/ are based on the comparison of the captured images.

By taking several images with the camera with a time interval in between, the gradual propagation of the conjugate through the porous membrane could be followed and a functional capillary flow could be confirmed.

In another implementation of the arrangement according to the first aspect, or any implementation thereof, the controller may be configured to evaluate performance of the milk dosing arrangement and/ or the lateral flow stick by generating a first index value, based on the colour or grey scale analysis of the first area, indicating: successfulness of application of the liquid sample to the sample pad; successfulness of capillar flow of the liquid along the longitudinal extension the porous membrane; and/ or release of conjugate from the conjugate pad to the liquid sample.

The first index, or dose index, may be set to a value between -100 and 100. The determined first index may be compared with a threshold limit, which may trigger an action. By studying a change of the first index of several lateral flow measurement over a time period, a degradation of the first index could be detected in time and an appropriate action may be triggered, such as for example cleaning or exchanging the tubes, hoses of the dosing modules, needle of the milk dosing arrangement that supply the milk sample, etc.

In another implementation of the arrangement according to the first aspect, or any implementation thereof, the arrangement may comprise a light source directed to illuminate the lateral flow stick when the image is captured. The milk dosing arrangement may be configured to prepare the liquid sample by mixing the obtained milk sample with a diluent before applying the liquid sample comprising milk mixed with diluent to the sample pad of the lateral flow stick.

Also, the controller may be configured to, in case the evaluation of the performance of the milk dosing arrangement indicates a capillar flow of the liquid through the porous membrane: define a third area of the image of the lateral flow stick substantially depicting a second subsection of the porous membrane; apply image processing on the third area of the image; and evaluate performance of the milk dosing arrangement, based on the applied image processing.

In another implementation of the arrangement according to the first aspect, or any implementation thereof, the third area of the image of the lateral flow stick may comprise at least one lateral edge of the lateral flow stick.

In another implementation of the arrangement according to the first aspect, or any implementation thereof, the controller may be configured to apply image processing on the third area of the image by a grey scale analysis of the third area. The grey scale analysis may comprise comparing intensity in the grey scale of the third area with a reference grey scale. The reference grey scale may be stored in a memory device communicatively connected to the controller. Also, the controller may evaluate performance of the milk dosing arrangement, based on the grey scale analysis of the third area of the image.

In case the liquid sample comprises too little milk due to technical failure during the testing process, in an extreme case only diluent, a reliable test result of the milk cannot be made, simply because there is no, or too little milk in the liquid sample for making a reliable test.

By estimating intensity in the grey scale, which corresponds to reflection and/ or absorption of light of the lateral flow stick in the third area of the image, it could be detected if the liquid sample comprises a sufficient amount of milk, or a milk/ diluent ratio exceeding a threshold limit, to enable a reliable lateral flow measurement based on the image processing.

The grey scale analysis may comprise determining a grey scale nuance, or intensity of the grey scale, of the third area. The determined grey scale nuance may be compared with a reference grey scale. Based on the outcome of the comparison, the amount of milk in the liquid sample, or the milk/ diluent ratio of the liquid sample is determined. The darker the grey scale nuance of the third area of the image is, the more milk is comprised in the liquid sample, in relation to the amount of diluent.

In another implementation of the arrangement according to the first aspect, or any implementation thereof, the controller may be configured to evaluate performance of the milk dosing arrangement by generating a second index value, or milk index. The second index value/ milk index may indicate: amount of milk in the mixture of milk and diluent in the liquid sample, i.e. the milk/ diluent ratio of the liquid sample.

In another implementation of the arrangement according to the first aspect, or any implementation thereof, the controller may be configured to perform an action for enhancing performance of the milk dosing arrangement and/ or the lateral flow stick, based on the applied image processing.

Thus, a detected low performance of the performance of the milk dosing arrangement and/ or the lateral flow stick, for example having resulted in a first index value, and/ or second index value lower than a respective threshold limit may trigger the performance enhancing action.

In another implementation of the arrangement according to the first aspect, or any implementation thereof, the action for enhancing performance of the milk dosing arrangement and/ or the lateral flow stick may comprise any one of: adjusting length of an incubation time for the lateral flow measurement, discarding the lateral flow stick, and/ or cleaning or exchanging of at least some elements of the milk dosing arrangement. The elements of the milk dosing arrangement to be cleaned may for example comprise tubes, hoses of the dosing modules, needle of the milk dosing arrangement that supply the milk sample, etc. The cleaning may be made by rinsing the element of the milk dosing arrangement with diluent or possibly some other cleaning liquid.

Thereby the performance enhancing action may be made upon detecting a degradation of the performance of the milk dosing arrangement and/ or the lateral flow stick, before the performance becomes so bad that the lateral flow measurement could not be used.

For example, when the first index value, and/ or the second index value is/ are lower than a respective threshold limit, the performance enhancing action may be triggered.

The farmer may thereby be alerted to change the lateral flow sticks before they have deteriorated so much that the result of the lateral flow test is affected. The farmer is released from the stress of continuously checking validity of the lateral flow sticks.

In another implementation of the arrangement according to the first aspect, or any implementation thereof, the action may comprise discarding the result of the liquid sample. The action may also comprise applying a new liquid sample comprising milk originating from the same animal as the discarded liquid sample, to be applied on a new lateral flow stick.

It is thereby possible to apply a new milk sample from the same animals to a new lateral flow stick, ensuring a continuous biomarker indication of milk samples from animals, also when there is a temporal disruption in the lateral flow measurement process, e.g. due to a single dysfunctional lateral flow stick.

Thanks to the described aspects, by determining validity of the result of the lateral flow test, the manual intervention, maintenance, worries and work intensity of the farmer is minimised or at least reduced.

Other advantages and additional novel features will become apparent from the subsequent detailed description.

### FIGURES

Embodiments of the invention will now be described in further detail with reference to the accompanying figures, in which:
- **Figure 1**: illustrates an example of an arrangement for measuring a biomarker value of a milk sample of an animal.
- **Figure 2A**: illustrates a lateral flow stick in a side view, according to an embodiment.
- **Figure 2B**: illustrates a lateral flow stick in a top view, according to an embodiment.
- **Figure 3**: illustrates a captured image of a lateral flow stick, according to an embodiment.
- **Figure 4**: illustrates elements of the arrangement for measuring a biomarker value of the animal milk sample in an embodiment.

### DETAILED DESCRIPTION

Embodiments of the invention described herein are defined as an arrangement, which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

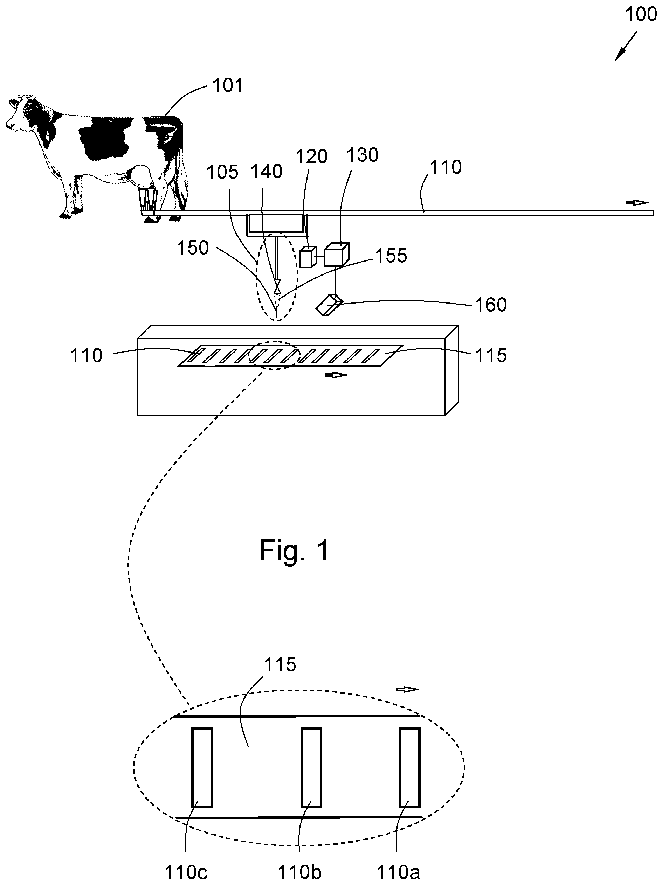

Figure 1 illustrates an arrangement 100. The arrangement 100 is arranged to evaluate performance of a milk dosing arrangement 105 and/ or a lateral flow stick 110 in association with extraction of a milk sample of an animal 101.

The animal 101 may be comprised in a herd of dairy animals at a dairy farm.

The lateral flow stick 110 is arranged to indicate a biomarker in a liquid sample comprising the milk sample extracted from the animal 101, by a lateral flow measurement.

"Animal" may be any arbitrary type of domesticated female milk producing mammal such as cow, goat, sheep, dairy buffalo, donkey, yak, etc.

Milk of the animal 101 may be extracted and evacuated by a milking equipment that are part of a milking parlour via a milk line to a milk tank or similar milk storage.

During milking of the animal 101, or in close relation therewith, the milk sample is diverted from the milking equipment/ milk line and provided to the milk dosing arrangement 105. The milk dosing arrangement 105 may comprise for example a tube element for attachment to an interface to the milking equipment/ milk line, a pump configured to act on the tube element, for forwarding the milk sample. The milk dosing arrangement 105 may also comprise a valve 140, a mixing chamber 155 and a needle 150 for receiving the milk sample via the tube element.

In some embodiments, the milk sample may be mixed with a diluent in the mixing chamber 155, thereby forming a liquid sample comprising a mixture of milk and diluent. The needle 150 of the milk dosing arrangement 105 may then be aligned with the lateral flow stick 110, where after the milk sample/ liquid sample is applied to the lateral flow stick 110 for a lateral flow measurement.

The control of the milk dosing arrangement 105, alignment of the lateral flow stick 110 with the needle 150 and application of the milk sample/ liquid sample to the lateral flow stick 110 may be made by a controller 130, with assistance from a visual input by a camera 120. The camera 120 is directed to capture an image of the lateral flow stick 110. Optionally, a light source 160 may be applied, to ensure proper illumination of the lateral flow stick 110 at least while the camera 120 is taking the image.

An advantage with applying a dedicated light source 160 comprised in the arrangement 100 is that it is ensured that there will be enough light when the camera 120 is taking the image of the lateral flow stick 110, for enabling a colour or grey scale analysis of the image.

The lateral flow stick 110 may be comprised on a carrier 115, comprising a plurality of lateral flow sticks 110a, 110b, 110c. The carrier 115 may be embodied e.g. as a carrier tape, or a blister package. In embodiments wherein the carrier 115 is embodied as a carrier tape, the carrier 115 may be comprised in a cassette.

The controller 130 is communicatively connected to the camera 120 and configured to generate an instruction to the camera 120, to capture an image of the lateral flow stick 110 at a predetermined time period after a point in time when the milk sample/ liquid sample has been applied to the lateral flow stick 110.

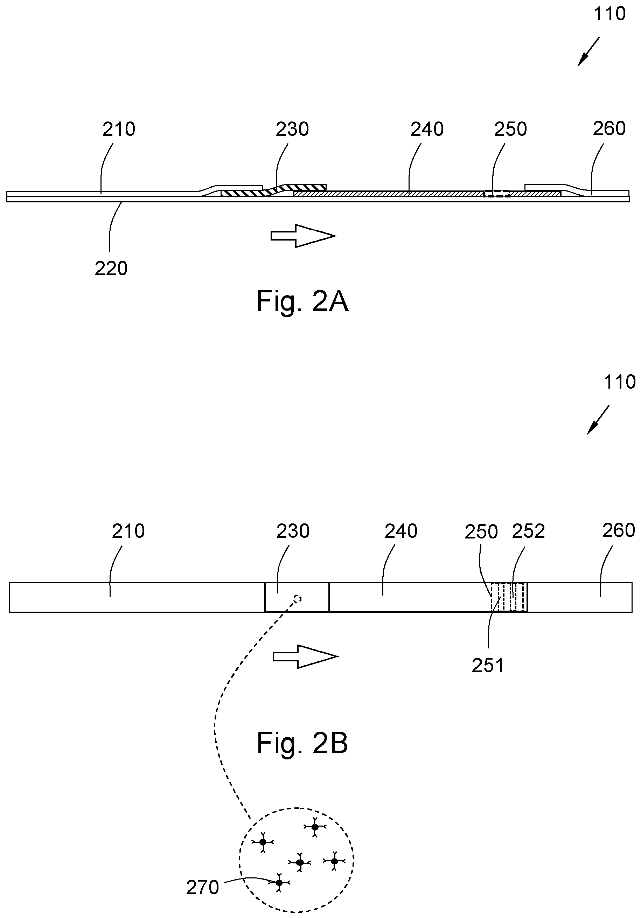

Figure 2A illustrates a lateral flow stick 110 as regarded from a side view while Figure 2B illustrates the lateral flow stick 110 as regarded from above.

The lateral flow stick 110 may comprise a back plate 220, which may be made of cardboard, paper or similar material. The back plate 220 may form a base on to which other components of the lateral flow stick 110 may be mounted.

The lateral flow stick 110 comprises a sample pad 210. The sample pad 210 is the part of the lateral flow stick 110 onto which the milk sample/ liquid sample is applied by the needle 150. The sample pad 210 may comprise a porous structure for enabling capillary flow such as cellulose fibres and/ or woven meshes.

The lateral flow stick 110 comprises a conjugate pad 230 comprising conjugate 270. The conjugate 270 may comprise antibody treated gold particles which are dispersed into the milk when the milk sample/ liquid sample is flowing from the sample pad 210 through the conjugate pad 230.

The conjugate 270 will react with the biomarker to be measured, for example progesterone in the milk of the applied liquid sample.

The conjugate 270 may thus be embedded in the conjugate pad 230 which may comprise a glass-fibre section of the lateral flow stick 110. Alternatively, the conjugate pad 230 may comprise cellulose and/ or surface modified polyester.

Also, the lateral flow stick 110 may comprise an indication zone 250, arranged to indicate the biomarker of the milk sample/ liquid sample, based on the conjugate 270. The lateral flow stick 110 may furthermore comprise a porous membrane 240 for receiving a capillary flow of the milk sample/ liquid sample from the conjugate pad 230, thereby forwarding conjugate 270 dispersed into the milk sample/ liquid sample to the indication zone 250.

The porous membrane 240 may comprise for example a nitrocellulose membrane, cellulose, glass fibre, polyester, rayon, a polymer, glass fibre, woven fibres, non-woven fibres, a chromatographic gel membrane, diatomaceous earth, silica gel, silicium oxide, kieselguhr, or other filtration membranes in different embodiments. The porous membrane 240 may be designed to enhance capillary pumping speed of liquid through the lateral flow stick 110. The indication zone 250 of the porous membrane 240 may comprise a test line 251 and a control line 252.

The test line 251 may be treated with a biomarker reference such as e.g. a progesterone reference which binds the antibody treated gold particles of the milk sample/ liquid sample and thereby brings the test line 251 to change colour tint when exposed for milk comprising a progesterone level lower than a threshold limit. Thus, the test line 251 may change colour tint into red/ reddish when the milk has no or low progesterone level. If/ when the animal 101 is in heat, the progesterone level is near zero in the milk sample. This colour change may then be detected by the camera 120 and may be reported to the farmer and/ or stored in a database associated with an identity and/ or a time reference of the animal 101. This may also trigger scheduling of insemination of the animal 101 (in case the biomarker is progesterone), or the scheduling of a veterinary check-up, for example (in case of another biomarker).

The control line 252 in the indication zone 250 of the porous membrane 240 may be treated with an antibody reference which binds antibody reference treated gold particles of the milk sample/ liquid sample regardless of the progesterone level in the milk, and may thereby bring the control line 252 to change colour tint when exposed for milk comprising antibody reference treated gold particles.

Thereby, in case the control line 252 has changed colour but the test line 251 has not, it typically is interpreted as: the milk sample/ liquid sample has been correctly applied and the conjugate 270 has been dispersed into the milk sample/ liquid sample but as the animal 101 is not in heat, the progesterone level of the milk of the animal 101 is lower than the threshold limit thereby causing the test line 251 to not change colour.

However, in case the control line 252 does not change colour tint when the milk/ liquid sample has been applied after an incubation time, the reason may be a dysfunction of either the lateral flow stick 110, or the milk dosing arrangement 105. The incubation time may be for example about 2-5 minutes.

The lateral flow stick 110, may comprise an absorbent pad 260. The absorbent pad 260 may comprise an absorbent configured to absorb superfluous milk/ liquid from the porous membrane 240.

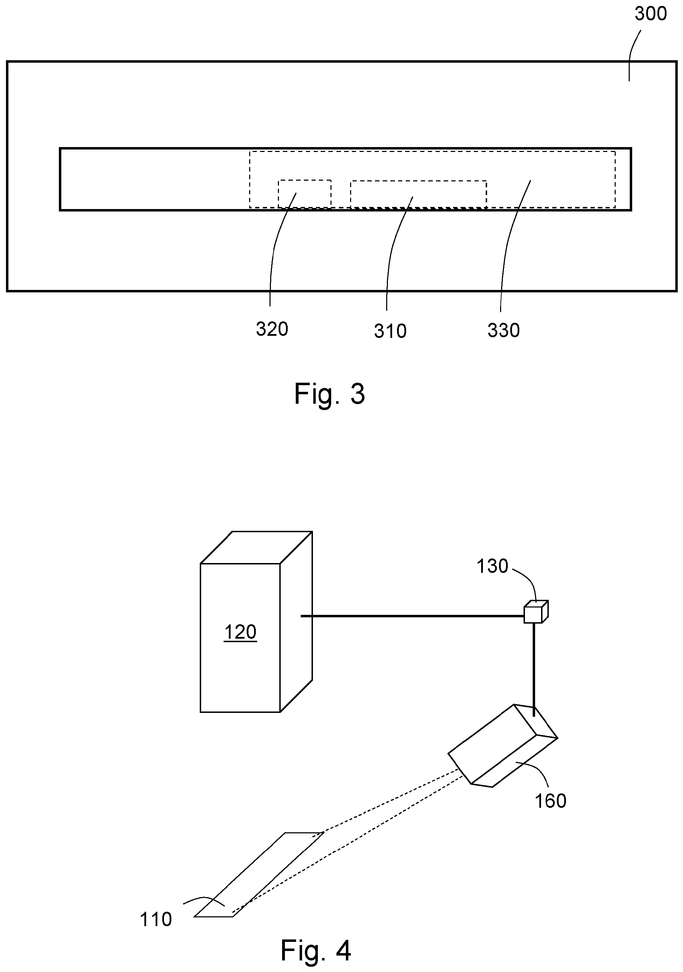

Figure 3 illustrates an image 300 of the lateral flow stick 110, captured by the camera 120.

The controller 130 is configured to generate an instruction to the camera 120, to capture an image 300 at a predetermined time period after a point in time when the liquid sample was applied to the lateral flow stick 110 via the needle 150.

The predetermined time period after the point in time when the liquid sample is applied to the lateral flow stick 110 may be shorter than the time it takes for the liquid sample to propagate by capillary flow from the sample pad 210 via the conjugate pad 230 and the porous membrane 240 to the indication zone 250 of the lateral flow stick 110. The predetermined time period may be for example 1-30 seconds, such as e.g. 6 seconds. The controller 130 then receives the captured image 300 from the camera 120.

The time it takes for the liquid sample to propagate by capillary flow from the sample pad 210 via the conjugate pad 230 and the porous membrane 240 to the indication zone 250 of the lateral flow stick 110 may be considerably longer, such as for example 2-5 minutes including the incubation time required for assuring that the test line 251 and/ or the control line 252 of the indication zone 250 has/ have sufficient time to change colour due to influence of the conjugate 270 dispersed into the milk/ liquid sample.

The controller 130 is configured to define a first area 310 of the image 300 of the lateral flow stick 110. The first area 310 is substantially depicting a subsection of the porous membrane 240 upstream the indication zone 250. Upstream in the current context means in the liquid flow direction of the applied liquid sample along the longitudinal extension of the lateral flow stick 110. Then, image processing is applied on the first area 310 of the image 300. The controller 130 is also configured to evaluate performance of the milk dosing arrangement 105 or the lateral flow stick 110, based on the applied image processing.

The controller 130 may be configured to perform the image processing by colour or grey scale analysis of the first area 310, and the camera 120 may be enabled to capture either a colour image, or a grey scale image.

The controller 130 may also be configured to perform the image processing and evaluate performance of the milk dosing arrangement 105 by comparing a colour or grey scale tint on the first area 310 of the captured image 300 with a reference colour or grey scale tint.

The reference colour or grey scale tint may be obtained from a first, i.e. previously taken image of the first area 310 captured before application of the liquid sample to the sample pad 210. In other embodiments, the reference colour or grey scale tint may be obtained from a database or memory.

The controller 130 is also configured to define a second area 320 of the image 300 of the lateral flow stick 110, substantially depicting a subsection of the conjugate pad 230.

The controller 130 is configured to estimate the colour or grey scale tint of the conjugate pad 230 on the first area 310 by comparing the colour or grey scale tint of the first area 310 with the colour or grey scale tint of the second area 320. Also, the controller 130 is configured to evaluate performance of the milk dosing arrangement 105 and/ or the lateral flow stick 110, based on the comparison of the colour or grey scale tint of the first area 310 with the colour or grey scale tint of the second area 320.

The second area 320 of the image 300 substantially covers a subsection of the area of the lateral flow stick 110 covering the conjugate pad 230 comprising the conjugate 270 while the first area 310 of the image 300 substantially covers the area of a subsection of the porous membrane 240 upstream the indication zone 250 in the liquid flow direction.

By comparing the colour or grey scale tint of the second area 320 with the colour or grey scale tint of the first area 310, release of conjugate 270 by the milk sample, or failure in release of conjugate 270 by the milk sample, respectively, is detected by the image processing of the controller 130.

The controller 130 may in some embodiments be configured to repeatedly generate instructions to the camera 120, to capture images 300 of the lateral flow stick 110, for example at a regular time interval. The controller 130 may then receive the captured images 300 from the camera 120 with a respective time stamp. For example, four images 300 may be taken, a first image 1 second before (t-1) applying the liquid sample (t), a second image 6 seconds after (t+6) having applied the liquid sample, a third image 12 seconds after (t+12) having applied the liquid sample, and a fourth image 18 seconds after (t+18) having applied the liquid sample, etc.

The image processing and the evaluation of the performance of the milk dosing arrangement 105 and/ or the lateral flow stick 110 made by the controller 130 may then be based on the comparison of the captured images 300.

The multitude of images 300 enable detection of flowing conjugate 270 through the lateral flow stick 110 by comparing colour or grey scale tint changes in different images 300, taken at different moments in time.

The controller 130 may also be configured to evaluate performance of the milk dosing arrangement 105 and/ or the lateral flow stick 110 by generating a first index value, based on the colour or grey scale analysis of the first area 310 and/ or the second area 320.

The first index value, or dose index may indicate: successfulness of application of the liquid sample to the sample pad 210; successfulness of capillar flow of the liquid along the longitudinal extension the porous membrane 240; and/ or release of conjugate 270 from the conjugate pad 230 to the liquid sample. The first index value may for example be scaled between -100 and 100 in some non-limiting embodiments.

The controller 130 may also be configured to define a third area 330 of the image 300 of the lateral flow stick 110 substantially depicting a second subsection of the porous membrane 240. The third area 330 may partially overlap at least the first area 310 and/ or the second area 320 in some embodiments. The third area 330 may also be larger than the first area 310 and/ or the second area 320. Preferably, the third area 330 may comprise at least one lateral edge of the lateral flow stick 110.

By applying the image analysis determining the intensity in grey scale close to the lateral edge of the lateral flow stick 110, it could be determined that the milk/ liquid sample as propagated all the way to the lateral edge of the lateral flow stick 110, thereby assuring a generous flow.

The controller 130 may define the third area 330 of the image 300 when the evaluation of the performance of the milk dosing arrangement 105, and/ or the lateral flow stick 110, indicates a capillar flow of the milk/ liquid through the porous membrane 240; and when diluent has been mixed with the milk to form the liquid sample.

Figure 4 is illustrating some details of the arrangement 100 such as the controller 130, the camera 120, and a light source 160.

The light source 160, which may be communicatively connected to the controller 130, may be directed to illuminate the lateral flow stick 110 when the image 300 is captured.

The controller 130 may then apply image processing on the third area 330 of the image 300, when having defined the third area 330 of the image 300 of the lateral flow stick 110.

The image processing performed by the controller 130 on the third area 330 may comprise estimation of reflection and/ or absorption of light from the light source 160 made by the surface of the part of the lateral flow stick 110 within the third area 330 of the image 300.

The reflection and/ or absorption of light may be estimated by a grey scale analysis of the third area 330, comparing intensity in the grey scale of the third area 330 with a reference grey scale. Different nuances of the reference grey scale may be associated with different levels of reflection and/ or absorption of light; and/ or a second index value, or milk index. The intensity in the grey scale of the image 300 may be compared with a reference grey scale, wherein different grey scale tints are associated with different grey scales. In general, the darker grey scale tint of the third area 330, the more milk in relation to diluent is comprised in the liquid sample.

The controller 130 may in addition be configured to evaluate performance of the milk dosing arrangement 105 and/ or the lateral flow stick 110, based on the estimated reflection and/ or absorption of light of the lateral flow stick 110, in the third area 330 of the image 300.

The purpose of estimating reflection and/ or absorption of light of the lateral flow stick 110 is to evaluate if the liquid sample comprises a sufficient amount of milk for making a reliable lateral flow test. As milk is an emulsion, or colloid, of butterfat globules within a water-based fluid, the reflection of light of the surface of the lateral flow stick 110 will be distinct from light reflection when the liquid sample comprises only diluent, or too little milk. Too little milk in this context means that the mixing ratio of the liquid sample of milk and diluent has a milk content smaller than a threshold level.

The controller 130 may generate a second index value, or milk index, indicating: amount of milk in the mixture of milk and diluent in the liquid sample. The second index value may for example have a value between 0 and 100 where 0 means no milk at all in the liquid sample, while 100 means no diluent (i.e. only milk) in the liquid sample. The performance of the milk dosing arrangement 105 and/ or the lateral flow stick 110 may thereby be made by the controller 130, based on the second index value.

The controller 130 may also be configured to perform an action for enhancing performance of the milk dosing arrangement 105 and/ or the lateral flow stick 110, based on the applied image processing. In case the first index value and/ or the second index value has/ have been generated, the action may be triggered when the first index value and/ or the second index value is lower than a predetermined trigger value.

The action to be performed may comprise for example adjusting length of an incubation time for the lateral flow measurement, discarding the lateral flow stick, and/ or cleaning at least some elements of the milk dosing arrangement 105 such as hoses and/ or pumps; and/ or rinsing the needle 150.

In case the evaluated performance of the lateral flow stick 110, based on the applied image processing, results in the conclusion that the milk dosing arrangement 105 and/ or the lateral flow stick 110 is dysfunctional, the lateral flow stick may be discarded. Possibly, also the farmer may be alerted, via an alert device.

In case the evaluated performance of the lateral flow stick 110, based on the applied image processing, results in the conclusion that the milk dosing arrangement 105 has not provided a liquid sample to the lateral flow stick 110, a cleaning action may be triggered. The cleaning action may comprise cleaning at least some elements of the milk dosing arrangement 105 such as hoses and/ or pumps; and/ or rinsing the needle 150.

In yet some embodiments, the action may comprise discarding the result of the liquid sample. Also, the action may comprise applying a new liquid sample comprising milk originating from the same animal 101 as the milk of the discarded liquid sample, to be applied on a new lateral flow stick 110.

Thus, a new lateral flow measurement may be made with milk from the same animal 101, already within some few seconds, for example 5-10 seconds, after having applied the first/ previous liquid sample.

At least some embodiments, or parts thereof, as illustrated in Figure 1, Figure 2A, Figure 2B, Figure 3 and/ or Figure 4 may with advantage be combined with each other for achieving further benefits.

The terminology used in the description of the embodiments as illustrated in the accompanying drawings is not intended to be limiting of the described arrangement 100. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware, but may also be distributed in other forms such as via Internet or other wired or wireless communication system.

## Claims

1. An arrangement (100), arranged to evaluate performance of a milk dosing arrangement (105) and/ or a lateral flow stick (110); wherein the arrangement (100) comprises:
the lateral flow stick (110) arranged to indicate a biomarker in a liquid sample comprising a milk sample extracted from an animal (101), by a lateral flow measurement; wherein the lateral flow stick (110) comprises
a sample pad (210) for receiving the liquid sample;
a conjugate pad (230) comprising
a conjugate (270) which is configured to react with the biomarker to be measured in the milk sample;
an indication zone (250), arranged to indicate the biomarker of the liquid sample, based on the conjugate (270);
a porous membrane (240) for receiving a capillary flow of the liquid sample from the conjugate pad (230), thereby forwarding conjugate (270) dispersed into the liquid sample to the indication zone (250);
the milk dosing arrangement (105), configured to
obtain a milk sample from the animal (101); and
apply the liquid sample comprising the milk sample to the sample pad (210) of the lateral flow stick (110);
a camera (120) directed to capture an image (300) of the lateral flow stick (110);
a controller (130), communicatively connected to the camera (120), wherein the controller (130) is configured to
generate an instruction to the camera (120), to capture an image (300) of the lateral flow stick (110) at a predetermined time period after a point in time when the liquid sample has been applied to the lateral flow stick (110);
receive the captured image (300) from the camera (120);
define a first area (310) of the image (300) of the lateral flow stick (110), substantially depicting a subsection of the porous membrane (240) upstream the indication zone (250) in the liquid flow direction of the applied liquid sample along the longitudinal extension of the lateral flow stick;
apply image processing on the first area (310) of the image (300);
**characterised in that** the controller (130) is further configured to define a second area (320) of the image (300) of the lateral flow stick (110), substantially depicting a subsection of the conjugate pad (230);
estimate the colour or grey scale tint of the conjugate pad (230) on the first area (310) by comparing the colour or grey scale tint of the first area (310) with the colour or grey scale tint of the second area (320); and
evaluate performance of the milk dosing arrangement (105) and/ or the lateral flow stick (110), based on the comparison of the colour or grey scale tint of the first area (310) with the colour or grey scale tint of the second area (320).

2. The arrangement (100) according to claim 1, wherein the controller (130) is configured to perform the image processing by colour or grey scale analysis of the first area (310).

3. **The** arrangement (100) according to any one of claim 1 or claim 2, wherein the controller (130) is configured to perform the image processing and evaluate performance of the milk dosing arrangement (105) by comparing a colour or grey scale tint on the first area (310) of the captured image (300) with a reference colour or grey scale tint.

4. **The** arrangement (100) according to claim 3, wherein the reference colour or grey scale tint is obtained from a first image of the first area (310) captured before application of the liquid sample to the sample pad (210).

5. **The** arrangement (100) according to any one of claim 1-4, wherein the predetermined time period after the point in time when the liquid sample is applied to the lateral flow stick (110) is shorter than the time it takes for the liquid sample to propagate by capillary flow from the sample pad (210) via the conjugate pad (230) and the porous membrane (240) to the indication zone (250) of the lateral flow stick (110), in addition to incubation time before a result is indicated by the indication zone (250).

6. The arrangement (100) according to any one of claim 1-5, wherein the controller (130) is configured to repeatedly
generate instructions to the camera (120), to capture images (300) of the lateral flow stick (110);
receive the captured images (300) from the camera (120) with a respective time stamp; and wherein the image processing and the evaluation of the performance of the milk dosing arrangement (105) and/ or the lateral flow stick (110) is/ are based on the comparison of the captured images (300).

7. The arrangement (100) according to any one of claim 2-6, wherein the controller (130) is configured to evaluate performance of the milk dosing arrangement (105) and/ or the lateral flow stick (110) by generating a first index value, based on the colour or grey scale analysis of the first area (310), indicating: successfulness of application of the liquid sample to the sample pad (210); successfulness of capillar flow of the liquid along the longitudinal extension the porous membrane (240); and/ or release of conjugate (270) from the conjugate pad (230) to the liquid sample.

8. The arrangement (100) according to any one of claims 1-7, comprising a light source (160) directed to illuminate the lateral flow stick (110) when the image (300) is captured; and wherein the milk dosing arrangement (105) is configured to prepare the liquid sample by mixing the obtained milk sample with a diluent before applying the liquid sample to the sample pad (210) of the lateral flow stick (110); and wherein
the controller (130) is configured to, in case the evaluation of the performance of the milk dosing arrangement (105) indicates a capillar flow of the liquid through the porous membrane (240):
define a third area (330) of the image (300) of the lateral flow stick (110) substantially depicting a second subsection of the porous membrane (240);
apply image processing on the third area (330) of the image (300); and
evaluate performance of the milk dosing arrangement (105), based on the applied image processing.

9. The arrangement (100) according to claim 8, wherein the third area (330) of the image (300) of the lateral flow stick (110) comprises at least one lateral edge of the lateral flow stick (110).

10. The arrangement (100) according to any one of claim 8 or claim 9, wherein the controller (130) is configured to
apply image processing on the third area (330) of the image (300) by a grey scale analysis of the third area (330), comparing intensity in the grey scale of the third area (330) with a reference grey scale; and
evaluate performance of the milk dosing arrangement (105), based on the grey scale analysis of the third area (330) of the image (300).

11. The arrangement (100) according to any one of claims 8-10, wherein the controller (130) is configured to evaluate performance of the milk dosing arrangement (105) by generating a second index value, indicating: amount of milk in the mixture of milk and diluent in the liquid sample.

12. The arrangement (100) according to any one of claim 1-11, wherein the controller (130) is configured to
perform an action for enhancing performance of the milk dosing arrangement (105) and/ or the lateral flow stick (110), based on the applied image processing.

13. The arrangement (100) according to claim 12, wherein the action comprises any one of: adjusting length of an incubation time for the lateral flow measurement, discarding the lateral flow stick (110), and/ or cleaning at least some elements of the milk dosing arrangement (105) such as hoses and/ or pumps and/ or rinse the needle (150) with diluent.

14. The arrangement (100) according to any one of claim 12 or claim 13, wherein the action comprises:
discarding the result of the liquid sample; and
applying a new liquid sample comprising milk originating from the same animal (101) as the discarded liquid sample, to be applied on a new lateral flow stick (110).

## Patentansprüche

1. Anordnung (100), die angeordnet ist, um Leistung einer Milchdosieranordnung (105) und/oder eines Lateral-Flow-Sticks (110) zu bewerten; wobei die Anordnung (100) umfasst:
den Lateral-Flow-Stick (110), der angeordnet ist, um einen Biomarker in einer Flüssigkeitsprobe, umfassend eine Milchprobe, die von einem Tier (101) extrahiert wird, durch eine Lateral-Flow-Messung anzugeben; wobei der Lateral-Flow-Stick (110) umfasst
ein Probenkissen (210) zum Aufnehmen der Flüssigkeitsprobe;
ein Konjugatkissen (230), umfassend ein Konjugat (270), das konfiguriert ist, um mit dem zu messenden Biomarker in der Milchprobe zu reagieren;
eine Angabezone (250), die angeordnet ist, um den Biomarker der Flüssigkeitsprobe basierend auf dem Konjugat (270) anzugeben;
eine poröse Membran (240) zum Aufnehmen eines Kapillarflusses der Flüssigkeitsprobe von dem Konjugatkissen (230), wobei dadurch Konjugat (270), das in die Flüssigkeitsprobe dispergiert ist, zu der Angabezone (250) weiterbewegt wird;
die Milchdosieranordnung (105), die konfiguriert ist, um eine Milchprobe von dem Tier (101) zu erhalten; und
die Flüssigkeitsprobe, umfassend die Milchprobe, auf das Probenkissen (210) des Lateral-Flow-Sticks (110) aufzubringen;
eine Kamera (120), die gerichtet ist, um ein Bild (300) des Lateral-Flow-Sticks (110) zu erfassen;
eine Steuervorrichtung (130), die mit der Kamera (120) kommunikativ verbunden ist, wobei die Steuervorrichtung (130) konfiguriert ist zum
Erzeugen einer Anweisung an die Kamera (120), ein Bild (300) des Lateral-Flow-Sticks (110) in einer zuvor bestimmten Zeitspanne nach einem Zeitpunkt, wenn die Flüssigkeitsprobe auf den Lateral-Flow-Stick (110) aufgebracht wurde, zu erfassen;
Empfangen des erfassten Bildes (300) von der Kamera (120);
Definieren eines ersten Bereichs (310) des Bildes (300) des Lateral-Flow-Sticks (110), der im Wesentlichen einen Teilabschnitt der porösen Membran (240) stromaufwärts der Angabezone (250) in der Flüssigkeitsflussrichtung der aufgebrachten Flüssigkeitsprobe entlang der Längserstreckung des Lateral-Flow-Sticks darstellt;
Anwenden von Bildverarbeitung auf den ersten Bereich (310) des Bildes (300);
**dadurch gekennzeichnet, dass** die Steuervorrichtung (130) ferner konfiguriert ist, um einen zweiten Bereich (320) des Bildes (300) des Lateral-Flow-Sticks (110), der im Wesentlichen einen Teilabschnitt des Konjugatkissens (230) darstellt, zu definieren;
Schätzen des Farb- oder Graustufentons des Konjugatkissens (230) auf dem ersten Bereich (310) durch Vergleichen des Farb- oder Graustufentons des ersten Bereichs (310) mit dem Farb- oder Graustufenton des zweiten Bereichs (320); und
Bewerten von Leistung der Milchdosieranordnung (105) und/oder des Lateral-Flow-Sticks (110) basierend auf dem Vergleich des Farb- oder Graustufentons des ersten Bereichs (310) mit dem Farb- oder Graustufenton des zweiten Bereichs (320).

2. Anordnung (100) nach Anspruch 1, wobei die Steuervorrichtung (130) konfiguriert ist, um die Bildverarbeitung durch Farb- oder Graustufenanalyse des ersten Bereichs (310) durchzuführen.

3. Anordnung (100) nach einem von Anspruch 1 oder 2, wobei die Steuervorrichtung (130) konfiguriert ist, um die Bildverarbeitung durchzuführen und Leistung der Milchdosieranordnung (105) durch Vergleichen eines Farb- oder Graustufentons auf dem ersten Bereich (310) des erfassten Bildes (300) mit einem Referenzfarb- oder Graustufenton zu bewerten.

4. Anordnung (100) nach Anspruch 3, wobei der Referenzfarb- oder Graustufenton aus einem ersten Bild des ersten Bereichs (310), das vor einer Aufbringung der Flüssigkeitsprobe auf das Probenkissen (210) aufgenommen wurde, erhalten wird.

5. Anordnung (100) nach einem der Ansprüche 1 bis 4, wobei die zuvor bestimmte Zeitspanne nach dem Zeitpunkt, wenn die Flüssigkeitsprobe auf den Lateral-Flow-Stick (110) aufgebracht wird, kürzer als die Zeit ist, die die Flüssigkeitsprobe benötigt, um sich durch Kapillarfluss von dem Probenkissen (210) über das Konjugatkissen (230) und die poröse Membran (240) zu der Angabezone (250) des Lateral-Flow-Sticks (110) auszubreiten, zusätzlich zu einer Inkubationszeit, bevor ein Ergebnis durch die Angabezone (250) angegeben wird.

6. Anordnung (100) nach einem der Ansprüche 1 bis 5, wobei die Steuervorrichtung (130) konfiguriert ist zum wiederholten
Erzeugen von Anweisungen an die Kamera (120), Bilder (300) des Lateral-Flow-Sticks (110) zu erfassen;
Empfangen der erfassten Bilder (300) von der Kamera (120) mit einem jeweiligen Zeitstempel; und wobei die Bildverarbeitung und die Bewertung der Leistung der Milchdosieranordnung (105) und/oder des Lateral-Flow-Sticks (110) auf dem Vergleich der erfassten Bilder (300) basiert/basieren.

7. Anordnung (100) nach einem der Ansprüche 2 bis 6, wobei die Steuervorrichtung (130) konfiguriert ist, um Leistung der Milchdosieranordnung (105) und/oder des Lateral-Flow-Sticks (110) durch Erzeugen eines ersten Indexwerts, basierend auf der Farb- oder Graustufenanalyse des ersten Bereichs, (310) zu bewerten, der angibt: Erfolg von Aufbringung der Flüssigkeitsprobe auf das Probenkissen (210); Erfolg von Kapillarfluss der Flüssigkeit entlang der Längserstreckung der porösen Membran (240); und/oder Freisetzung von Konjugat (270) aus dem Konjugatkissen (230) zu der Flüssigkeitsprobe.

8. Anordnung (100) nach einem der Ansprüche 1 bis 7, umfassend eine Lichtquelle (160), die gerichtet ist, um den Lateral-Flow-Stick (110) zu beleuchten, wenn das Bild (300) erfasst wird; und wobei die Milchdosieranordnung (105) konfiguriert ist, um die Flüssigkeitsprobe durch Mischen der erhaltenen Milchprobe mit einem Verdünnungsmittel herzustellen, bevor die Flüssigkeitsprobe auf das Probenkissen (210) des Lateral-Flow-Sticks (110) aufgebracht wird; und wobei
die Steuervorrichtung (130) konfiguriert ist, um, falls die Bewertung der Leistung der Milchdosieranordnung (105) einen Kapillarfluss der Flüssigkeit durch die poröse Membran (240) angibt:
einen dritten Bereich (330) des Bildes (300) des Lateral-Flow-Sticks (110), der im Wesentlichen einen zweiten Teilabschnitt der porösen Membran (240) darstellt, zu definieren;
Bildverarbeitung auf den dritten Bereich (330) des Bildes (300) anzuwenden; und
Leistung der Milchdosieranordnung (105) basierend auf der angewandten Bildverarbeitung zu bewerten.

9. Anordnung (100) nach Anspruch 8, wobei der dritte Bereich (330) des Bildes (300) des Lateral-Flow-Sticks (110) mindestens einen lateralen Rand des Lateral-Flow-Sticks (110) umfasst.

10. Anordnung (100) nach einem der Ansprüche 8 oder 9, wobei die Steuervorrichtung (130) konfiguriert ist zum
Anwenden von Bildverarbeitung auf den dritten Bereich (330) des Bildes (300) durch eine Graustufenanalyse des dritten Bereichs (330), wobei eine Intensität in der Graustufe des dritten Bereichs (330) mit einer Referenzgraustufe verglichen wird; und
Bewerten von Leistung der Milchdosieranordnung (105) basierend auf der Graustufenanalyse des dritten Bereichs (330) des Bildes (300).

11. Anordnung (100) nach einem der Ansprüche 8 bis 10, wobei die Steuervorrichtung (130) konfiguriert ist, um Leistung der Milchdosieranordnung (105) durch Erzeugen eines zweiten Indexwerts zu bewerten, der angibt: Menge an Milch in der Mischung aus Milch und Verdünnungsmittel in der Flüssigkeitsprobe.

12. Anordnung (100) nach einem der Ansprüche 1 bis 11, wobei die Steuervorrichtung (130) konfiguriert ist zum
Durchführen einer Aktion zum Verbessern von Leistung der Milchdosieranordnung (105) und/oder des Lateral-Flow-Sticks (110) basierend auf der angewendeten Bildverarbeitung.

13. Anordnung (100) nach Anspruch 12, wobei die Aktion ein beliebiges umfasst von: Anpassen der Länge einer Inkubationszeit für die Lateral-Flow-Messung, Verwerfen des Lateral-Flow-Sticks (110) und/oder Reinigen mindestens einiger Elemente der Milchdosieranordnung (105), wie Schläuche und/oder Pumpen, und/oder Spülen der Nadel (150) mit Verdünnungsmittel.

14. Anordnung (100) nach einem der Ansprüche 12 oder 13, wobei die Aktion umfasst:
Verwerfen des Ergebnisses der Flüssigkeitsprobe; und
Aufbringen einer neuen Flüssigkeitsprobe, umfassend Milch, die von demselben Tier (101) wie die verworfene Flüssigkeitsprobe stammt, die auf einen neuen Lateral-Flow-Stick (110) aufzubringen ist.

## Revendications

1. Un agencement (100), agencé pour évaluer les performances d'un agencement de dosage de lait (105) et/ou d'un bâtonnet de flux latéral (110) ; et dans lequel l'agencement (100) comprend :
le bâtonnet de flux latéral (110) agencé pour indiquer un biomarqueur dans un échantillon liquide comprenant un échantillon de lait extrait d'un animal (101), par une mesure de flux latéral ; dans lequel le bâtonnet de flux latéral (110) comprend
un tampon d'échantillon (210) pour recevoir l'échantillon liquide ;
un tampon conjugué (230) comprenant un conjugué (270) qui est conçu pour réagir avec le biomarqueur à mesurer dans l'échantillon de lait ;
une zone d'indication (250), agencée pour indiquer le biomarqueur de l'échantillon liquide, en fonction du conjugué (270) ;
une membrane poreuse (240) pour recevoir un flux capillaire de l'échantillon liquide à partir du tampon conjugué (230), acheminant de ce fait le conjugué (270) dispersé dans l'échantillon liquide vers la zone d'indication (250) ;
l'agencement de dosage de lait (105), conçu pour obtenir un échantillon de lait de l'animal (101) ; et
l'application de l'échantillon liquide comprenant l'échantillon de lait sur le tampon d'échantillon (210) du bâtonnet de flux latéral (110) ;
une caméra (120) dirigée pour capturer une image (300) du bâtonnet de flux latéral (110) ;
un dispositif de commande (130), connecté en communication à la caméra (120), dans lequel le dispositif de commande (130) est configuré pour
générer une instruction à la caméra (120), afin de capturer une image (300) du bâtonnet de flux latéral (110) à un laps de temps prédéterminé après un instant dans le temps où l'échantillon liquide a été appliqué sur le bâtonnet de flux latéral (110) ;
recevoir l'image capturée (300) à partir de la caméra (120) ;
définir une première zone (310) de l'image (300) du bâtonnet de flux latéral (110), représentant essentiellement une sous-section de la membrane poreuse (240) en amont de la zone d'indication (250) dans la direction du flux de liquide de l'échantillon liquide appliqué le long de l'extension longitudinale du bâtonnet de flux latéral ;
appliquer un traitement d'image sur la première zone (310) de l'image (300) ;
**caractérisé en ce que** le dispositif de commande (130) est en outre configuré pour définir une deuxième zone (320) de l'image (300) du bâtonnet de flux latéral (110) représentant essentiellement une sous-section du tampon conjugué (230) ;
estimer la teinte de couleur ou d'échelle de gris du tampon conjugué (230) sur la première zone (310) en comparant la teinte de couleur ou d'échelle de gris de la première zone (310) avec la teinte de couleur ou d'échelle de gris de la deuxième zone (320) ; et
évaluer les performances de l'agencement de dosage de lait (105) et/ou du bâtonnet de flux latéral (110), en fonction de la comparaison de la teinte de couleur ou d'échelle de gris de la première zone (310) avec la teinte de couleur ou d'échelle de gris de la deuxième zone (320).

2. Agencement (100) selon la revendication 1, dans lequel le dispositif de commande (130) est configuré pour réaliser le traitement d'image par analyse de couleur ou d'échelle de gris de la première zone (310).

3. Agencement (100) selon l'une quelconque de la revendication 1 ou la revendication 2, dans lequel le dispositif de commande (130) est configuré pour réaliser le traitement d'image et évaluer les performances de l'agencement de dosage de lait (105) en comparant une teinte de couleur ou d'échelle de gris sur la première zone (310) de l'image capturée (300) avec une teinte de couleur de référence ou d'échelle de gris.

4. Agencement (100) selon la revendication 3, dans lequel la teinte de couleur de référence ou d'échelle de gris est obtenue à partir d'une première image de la première zone (310) capturée avant l'application de l'échantillon liquide sur le tampon d'échantillon (210).

5. Agencement (100) selon l'une quelconque des revendications 1 à 4, dans lequel le laps de temps prédéterminé après l'instant dans le temps où l'échantillon liquide est appliqué sur le bâtonnet de flux latéral (110) est plus court que le temps qu'il faut à l'échantillon liquide pour se propager par flux capillaire du tampon d'échantillon (210) par l'intermédiaire du tampon conjugué (230) et de la membrane poreuse (240) jusqu'à la zone d'indication (250) du bâtonnet de flux latéral (110), en plus du temps d'incubation avant qu'un résultat ne soit indiqué par la zone d'indication (250).

6. Système (100) selon l'une quelconque des revendications 1 à 5, dans lequel le dispositif de commande (130) est configuré pour, de manière répétée,
générer des instructions destinées à la caméra (120), afin de capturer des images (300) du bâtonnet de flux latéral (110) ;
recevoir les images capturées (300) provenant de la caméra (120) avec un horodatage respectif ; et dans lequel le traitement d'image et l'évaluation des performances de l'agencement de dosage de lait (105) et/ou du bâtonnet de flux latéral (110) est/sont basé(s) sur la comparaison des images capturées (300).

7. Agencement (100) selon l'une quelconque des revendications 2 à 6, dans lequel le dispositif de commande (130) est configuré pour évaluer les performances de l'agencement de dosage de lait (105) et/ou du bâtonnet de flux latéral (110) en générant une première valeur d'indice, en fonction de l'analyse de couleur ou d'échelle de gris de la première zone (310), indiquant : la réussite de l'application de l'échantillon liquide sur le tampon d'échantillon (210) ; la réussite du flux capillaire du liquide le long de l'extension longitudinale de la membrane poreuse (240) ; et/ou la libération de conjugué (270) à partir du tampon conjugué (230) vers l'échantillon liquide.

8. Agencement (100) selon l'une quelconque des revendications 1 à 7, comprenant une source lumineuse (160) dirigée pour éclairer le bâtonnet de flux latéral (110) lorsque l'image (300) est capturée ; et dans lequel l'agencement de dosage de lait (105) est conçu pour préparer l'échantillon liquide en mélangeant l'échantillon de lait obtenu avec un diluant avant d'appliquer l'échantillon liquide sur le tampon d'échantillon (210) du bâtonnet de flux latéral (110) ; et dans lequel
le dispositif de commande (130) est configuré pour, dans le cas où l'évaluation des performances de l'agencement de dosage de lait (105) indique un flux capillaire du liquide à travers la membrane poreuse (240) :
définir une troisième zone (330) de l'image (300) du bâtonnet de flux latéral (110) représentant essentiellement une seconde sous-section de la membrane poreuse (240) ;
appliquer un traitement d'image sur la troisième zone (330) de l'image (300) ; et
évaluer les performances de l'agencement de dosage de lait (105), sur la base du traitement d'image appliqué.

9. Agencement (100) selon la revendication 8, dans lequel la troisième zone (330) de l'image (300) du bâtonnet de flux latéral (110) comprend au moins un bord latéral du bâtonnet de flux latéral (110).

10. Agencement (100) selon l'une quelconque de la revendication 8 ou de la revendication 9, dans lequel le dispositif de commande (130) est configuré pour
appliquer un traitement d'image sur la troisième zone (330) de l'image (300) par une analyse de l'échelle de gris de la troisième zone (330), en comparant une intensité dans l'échelle de gris de la troisième zone (330) avec une échelle de gris de référence ; et
évaluer les performances de l'agencement de dosage de lait (105), en fonction de l'analyse de l'échelle de gris de la troisième zone (330) de l'image (300).

11. Agencement (100) selon l'une quelconque des revendications 8 à 10, dans lequel le dispositif de commande (130) est configuré pour évaluer les performances de l'agencement de dosage de lait (105) en générant une seconde valeur d'indice, indiquant : une quantité de lait dans le mélange de lait et de diluant dans l'échantillon liquide.

12. Agencement (100) selon l'une quelconque des revendications 1 à 11, dans lequel le dispositif de commande (130) est configuré pour
mettre en œuvre une action permettant d'améliorer les performances de l'agencement de dosage de lait (105) et/ou du bâtonnet de flux latéral (110), sur la base du traitement d'image appliqué.

13. Agencement (100) selon la revendication 12, dans lequel l'action comprend l'une quelconque parmi : l'ajustement de la longueur d'un temps d'incubation pour la mesure de flux latéral, l'élimination du bâtonnet de flux latéral (110), et/ou le nettoyage d'au moins certains éléments de l'agencement de dosage de lait (105) tels que des tuyaux et/ou des pompes et/ou le rinçage de l'aiguille (150) avec un diluant.

14. Agencement (100) selon l'une quelconque de la revendication 12 ou de la revendication 13, dans lequel l'action comprend :
rejeter le résultat de l'échantillon liquide ; et
l'application d'un nouvel échantillon liquide comprenant du lait provenant du même animal (101) en guise de l'échantillon liquide éliminé, à appliquer sur un nouveau bâtonnet de flux latéral (110).
